(19) Europäisches Patentamt
European Patent Office
Office européen des brevets

(11) Veröffentlichungsnummer: **0 075 201**
**B1**

(12) # EUROPÄISCHE PATENTSCHRIFT

(45) Veröffentlichungstag der Patentschrift: **10.07.85**

(51) Int. Cl.[4]: **C 07 C 17/28**, C 07 C 19/02

(21) Anmeldenummer: **82108285.6**

(22) Anmeldetag: **09.09.82**

(54) **Verfahren zur Herstellung von 1,1,1-Trichlormethylverbindungen.**

(30) Priorität: **17.09.81 DE 3136983**

(43) Veröffentlichungstag der Anmeldung:
**30.03.83 Patentblatt 83/13**

(45) Bekanntmachung des Hinweises auf die Patenterteilung:
**10.07.85 Patentblatt 85/28**

(84) Benannte Vertragsstaaten:
**BE CH DE FR GB LI**

(56) Entgegenhaltungen:
**BE - A - 622 938**
**GB - A - 2 043 071**
**US - A - 3 213 149**

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft, Carl-Bosch-Strasse 38, D-6700 Ludwigshafen (DE)**

(72) Erfinder: **Fischer, Martin, Dr., Elbingerweg 1, D-6700 Ludwigshafen 29 (DE)**

**EP 0 075 201 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

## Beschreibung

Die Erfindung betrifft ein Verfahren zur Herstellung von 1,1,1-Trichlormethylverbindungen durch Umsetzung von Chloroform mit Olefinen in Gegenwart von Radikalstartern und in Gegenwart von schwachbasischen Alkali- oder Erdalkalisalzen.

Es ist bekannt, daß bei der Umsetzung von Chloroform mit 1-Octen in Gegenwart von Benzoylperoxid 1,1,1-Trichlornonan mit Selektivitäten von 41% bis 48% bei unvollständigen Olefinumsätzen entsteht (Ausbeuten von 22 bis 40%, bezogen auf Olefin) (JACS 69, 1100 bis 1104 [1947], insbesondere Seite 1104; J. Chem. Soc. 1963, 3921 bis 3927, insbesondere Seite 3927). Auch die radikalischen Chloroformadditionen an andere Olefine verlaufen im allgemeinen mit nur mäßigen Ausbeuten (C. Walling und E. S. Hugser, Org. Reactions 13, 129 [1963]).

Um die mit der radikalischen Addition von Chloroform an C—C-Doppelbindungen konkurrierende Polymerisation des olefinischen Partners zu unterdrücken, ist es günstig, einen möglichst hohen Überschuß an Chloroform anzuwenden. In der DE-OS 2 616 528 wird in Beispiel 1 gezeigt, daß die Ausbeute an 1,1,1-Trichlor-4-methyl-4-pentanol 69% sein soll, wenn die Ausgangsstoffe Chloroform und Dimethylvinylcarbinol im Molverhältnis 9,4 : 1 eingesetzt werden. Für gute Raum-Zeit-Ausbeuten und geringe Aufarbeitungskosten ist natürlich ein möglichst niedriger Chloroformüberschuß anzustreben. Da bei der Radikaladdition von Chloroform an Olefine Spuren von Chlorwasserstoff freigesetzt werden, kann die Umsetzung nicht in Eisen-haltigen Reaktionsgefäßen durchgeführt werden. Durch HCl wird nicht nur das Reaktionsgefäß angegriffen. Spuren von Eisenionen im Reaktionsgemisch führen außerdem zu unerwünschten Nebenreaktionen, z. B. zur verstärkten Abspaltung von HCl aus den Reaktionsprodukten, zu Chloratomübertragungen und zur Wasserabspaltung, wenn der Ausgangsstoff II OH-Gruppen enthält. Diese Komplikationen können durch Arbeiten in Glas- oder Emailgefäßen zwar vermieden werden. Druckbeständige, mit Glas oder Email ausgekleidete Reaktoren sind jedoch wesentlich teurer als solche aus Stahl.

US-A-3 213 149 beschreibt die Umsetzung von Telogenen wie Tetrachlorkohlenstoff, Chloroform, Alkoholen, Aldehyden und Essigsäure mit Olefinen zu Telomeren in Gegenwart von Salzen organischer Säuren und gegebenenfalls Aminen. Spalte 1, Zeile 50 beschreibt ausdrücklich ein Verfahren, das keine organischen Peroxide als Radikalstarter benötigt (Beispiele). Wesentliches Merkmal des Verfahrens ist gerade der Ersatz der Peroxide durch Metallsalze als Katalysatoren (Spalte 1, Zeilen 35 bis 50, insbesondere Zeilen 49 und 50; Spalte 2, Zeilen 33 bis 34). Die erhaltenen Telogene sind durchweg Produktgemische. Endstoffe, in die nur ein einziges Olefin eingebaut wurde, entstehen nur in untergeordnetem Maße. Chlorform als Telogen wird nicht durch Beispiele gezeigt.

Im Falle der Addition von Tetrachlorkohlenstoff handelt es sich um folgende Additionsreaktion (n = Gemisch)

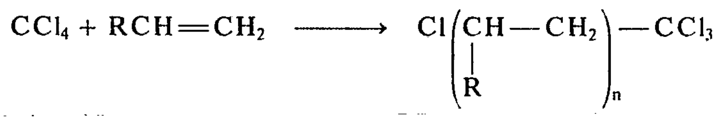

Aus Spalte 1, Zeile 18 ist zu entnehmen, daß bei Einsatz von Chloroform die folgende analoge Umsetzung erfolgt:

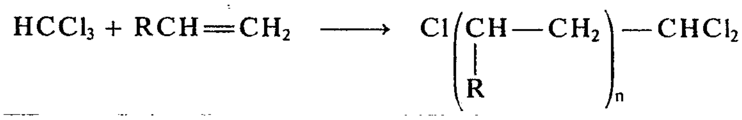

Unter Chlorübertragung verlaufende Telomerisierungen mit Chloroform als Telogen, die durch Übergangsmetallionen katalysiert werden, sind aus J. Chem. Soc. 1963, 3921, bekannt.

Alkali- und Erdalkaliverbindungen werden nicht bevorzugt, aber ausdrücklich die Eisen- und Kupfersalze herausgestellt (Spalte 1, Zeilen 62—64). Die Beispiele, die Alkali- und Erdalkalisalze verwenden (26, 27, 29, 30, 31) zeigen im Vergleich, z. B. Beispiel 38, daß die Aktivität von Alkalisalzen auch bei der Telomerisierung von Tetrachlorkohlenstoff im Vergleich zu Übergangsmetallen unbefriedigend ist. Als wesentliches Merkmal wird weiterhin die Verwendung von Aminen, die in Kombination mit den Metallsalzen einen wesentlichen katalytischen Effekt geben (Sp. 1, Z. 68—71; Sp. 2, Z. 7—12 und 25—26), beschrieben. Außer Beispiel 4 (mit unbefriedigendem Ergebnis) werden in allen Beispielen Amine verwendet.

Es wurde nun gefunden, daß man 1,1,1-Trichlormethylverbindungen der Formel

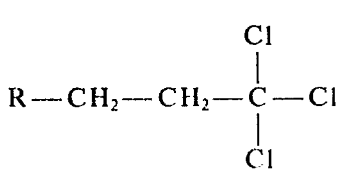

(I)

worin R einen aliphatischen Rest bedeutet, durch Umsetzung von Chloroform mit Olefinen der Formel

$$R-CH=CH_2 \qquad (II)$$

worin R die vorgenannte Bedeutung besitzt, in Gegenwart von Radikalstartern vorteilhaft erhält, wenn die Umsetzung in Gegenwart von schwachbasischen Alkali- oder Erdalkalisalzen durchgeführt wird.

Die Umsetzung kann im Falle der Verwendung von 3-Methylbuten-1-ol-3 durch die folgenden Formeln wiedergegeben werden:

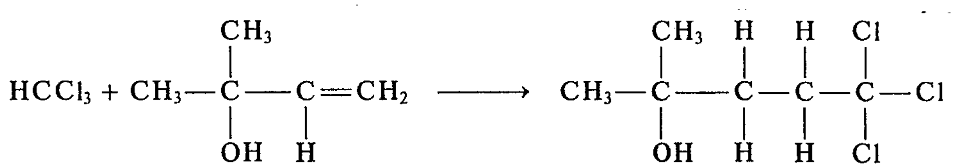

Im Vergleich zu den bekannten Verfahren liefert das Verfahren nach der Erfindung auf einfacherem und wirtschaftlicherem Wege 1,1,1-Trichlormethylverbindungen in besserer Ausbeute. So steigt beispielsweise die Ausbeute an 1,1,1-Trichlornonan von 59% auf 72% an, wenn dem Reaktionsgemisch aus Chloroform, 1-Octen und tert.-Butylperbenzoat 8 Gew.-% (bezogen auf Olefin) wasserfreies Natriumacetat zugesetzt werden. Die Ausbeuten bei dem erfindungsgemäßen Verfahren sind auch wesentlich höher als die Ausbeuten der in US-A-3 213 149 beschriebenen strukturell andersartigen Telogene mit $n = 1$. Telomere mit $n = 2$ oder mehr Alkylengruppen treten bei dem erfindungsgemäßen Verfahren nicht in wesentlichen Mengen auf. Durch den erfindungsgemäßen Zusatz schwachbasischer Alkali- oder Erdalkalisalze ist es möglich, die Chlorformaddition in Stahlgefäßen durchzuführen, ohne daß Eisen aus dem Wandmaterial herausgelöst wird.

Bei dem erfindungsgemäßen Verfahren wird nicht Chlor, sondern Wasserstoff auf das Olefin übertragen,

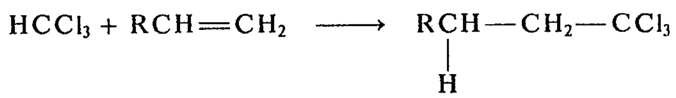

d. h. es handelt sich nicht um ein Verfahren zur Herstellung von 1,1,3-Trichlorverbindungen noch um eine Telomerisierung. Es war daher im Hinblick auf US-A-3 213 149 überraschend, daß das erfindungsgemäße Verfahren anstelle von 1,1,3-Trichlorverbindungen 1,1,1-Trichlorverbindungen liefert, ebenso unerwartet war die Bildung von monomeren Endstoffen und noch dazu in hohen Ausbeuten anstelle von Telomeren und Telomerengemischen (s. a. Vergleichsbeispiele 1 bis 4).

Das erfindungsgemäße Verfahren liefert monomere 1,1,1-Verbindungen als Endstoffe und noch dazu in hoher Ausbeute und Reinheit.

Die Ausgangsstoffe können in stöchiometrischer Menge oder im Überschuß, zweckmäßig in einem Verhältnis von 2 bis 10, insbesondere 4 bis 8 Mol Chloroform je Mol Ausgangsstoff II, umgesetzt werden. Ein Lösungsmittel ist nicht erforderlich. Bevorzugte Ausgangsstoffe II und dementsprechend bevorzugte Endstoffe I sind solche, in deren Formeln R einen Alkylrest mit 1 bis 20, insbesondere 1 bis 8 Kohlenstoffatomen bedeutet, der unsubstituiert oder mit OH-Gruppen und/oder Chloratomen, vorzugsweise 1 oder 2 OH-Gruppen und/oder Chloratomen, substituiert sein kann.

So kommen beispielsweise als Ausgangsstoffe II in Frage:

1-Propen, 1-Buten, Isobutylen, 1-Penten, 1-Hexen, 1-Hepten, 1-Octen, 1-Nonen, 1-Decen, 1-Dodecen; Propen-2-ol-1, 3-Methylbuten-1, 3-Methylbuten-1-ol-3, 3-Chlorpropen-1, 3-Chlorbuten-1, 3,4-Dichlorbuten-1, Buten-1-ol-3, Buten-1-diol-3,4.

Unter schwachbasischen Salzen sind alle die Salze zu verstehen, die geeignet sind, Chlorwasserstoff unter Salzbildung abzufangen. Zweckmäßig nimmt man Alkali- oder Erdalkalisalze von Säuren mit Dissoziationskonstanten $\leqslant 10^{-3}$.

Geeignete schwachbasische Salze sind beispielsweise die Alkali- oder Erdalkalisalze organischer Säuren wie Ameisensäure, Essigsäure, Propionsäure, Buttersäure, Malonsäure, Bernsteinsäure, Maleinsäure oder Benzoesäure. Weiterhin sind die basischen Salze mehrbasischer Mineralsäuren wie Phosphorsäure, Borsäure, phosphorige Säure, schweflige Säure oder Kieselsäure geeignet. Geeignet sind außerdem Alkali- und Erdalkalicarbonate und -hydrogencarbonate. Bevorzugte Salze sind Magnesium-, Calcium-, Lithium- und insbesondere Natrium- und Kaliumsalze.

Die schwachbasischen Salze werden in Mengen von 1 bis 20 Gew.-%, bezogen auf Ausgangsstoff II, eingesetzt. Bevorzugt werden Mengen von 3 bis 12 Gew.-%.

Unter Radikalstarter werden hier Initiatoren und Katalysatoren, die polymerisationsauslösende oder polymerisationsbeschleunigende Zusätze sind, verstanden. Bezüglich der Definition werden auf Houben—Weyl, Methoden der Org. Chemie, Band 14/1, Seite 56, verwiesen.

Als Radikalstarter für die Chloroformaddition verwendet man die für den Start von Radikalkettenreaktionen üblichen Initiatoren:

Dialkylperoxide wie Di-tert.-butylperoxide oder 2,5-Dimethyl-2,5-bis-(tert.-butylperoxi)-hexan; Perester wie tert.-Butyl-peroxipivalat, tert.-Butylperoxioctoat, tert.-Butylperoxiisobutyrat, tert.-Butylperoximaleinsäure, 2,5-Dimethylhexan-2,5-diperbenzoat, tert.-Butylperacetat, Di-tert.-butyldiperphthalat oder tert.-Butylperbenzoat; Diacylperoxide wie Pelargonylperoxid, Decanoylperoxid, Lauroylperoxid, Propionylperoxid, Acetylperoxid, Benzoylperoxid oder p-Chlorbenzoylperoxid; Hydroperoxide wie tert.-Butylhydroperoxid, Cumolhydroperoxid oder Ketonhydroperoxide; Peroxidicarbonate wie Bis-(4-tert.-butylcyclohexyl)-peroxidicarbonat, Dicyclohexylperoxidicarbonat, Bis-(2-ethyl-hexyl)-peroxidicarbonat, Dibutylperoxidicarbonat, Diisopropyl-peroxidicarbonat.

Die Menge Radikalstarter, die für die Addition von Chloroform an die ungesättigten Ausgangsstoffe II benötigt wird, hängt von der Struktur der Ausgangsstoffe II, der Temperatur und der Struktur des verwendeten Starters ab. Man verwendet zweckmäßig 1 bis 10 Mol-%, bevorzugt 3 bis 7 Mol-% Peroxid, bezogen auf Ausgangsstoff II.

Die Umsetzung wird im allgemeinen bei einer Temperatur von 60 bis 180°C, bevorzugt von 110 bis 150°C, drucklos oder unter Druck, kontinuierlich oder diskontinuierlich durchgeführt. Die Reaktionszeiten richten sich nach der Halbwertszeit des eingesetzten Radikalstarters. Für eine gute Ausnutzung des Initiators ist es zweckmäßig, die Reaktionszeit auf das 3- bis 6fache der Halbwertszeit des Peroxids bei der vorgewählten Temperatur festzulegen. Längere Reaktionszeiten schaden jedoch in den Fällen, in denen die Ausgangsstoffe II thermisch stabil sind, nicht. Zweckmäßig wird man die Temperatur und den Starter so wählen, daß die Reaktionszeiten auf 0,5 bis 24 h, bevorzugt auf 2 bis 8 h, festgelegt werden können.

Die Reaktion kann wie folgt durchgeführt werden: Ein Gemisch von Chloroform, schwachbasischem Salz, Ausgangsstoff II und Radikalstarter wird bei der Reaktionstemperatur während vorgenannter Reaktionszeit gehalten. Der Endstoff wird dann in üblicher Weise, z. B. durch Zusatz von Wasser und fraktionierte Destillation der organischen Phase, isoliert.

Da radikalische Chloroformadditionen stark exotherm verlaufen, ist es bei Durchführung der Reaktionen im technischen Maßstab jedoch sicherheitstechnisch vorteilhaft, zumindest die Ausgangsstoffe II nach und nach in das Reaktionsgefäß einzudosieren. Um ein über die Reaktionszeit gleichmäßiges Angebot an Startradikalen zu schaffen, ist es zweckmäßig, auch den Radikalstarter zuzudosieren. Die Zudosierzeiten von Ausgangsstoff II und Radikalstarter betragen vorteilhaft 1/3 bis 2/3 der Gesamtreaktionszeiten. Nach Beendigung der Reaktion kann das basische Salz abfiltriert oder in Wasser gelöst und mit der wäßrigen Phase abgetrennt werden. Das bei der Reaktion nicht verbrauchte Chloroform wird abdestilliert. Der dabei im Rückstand verbleibende Endstoff I kann durch Kristallisation oder bevorzugt durch Vakuumdestillation isoliert werden.

Die nach dem erfindungsgemäßen Verfahren herstellbaren 1,1,1-Trichlormethylverbindungen sind wertvolle Vorprodukte für die Herstellung von Farbstoffen, Pharmazeutika und Schädlingsbekämpfungsmitteln. Sie sind Ausgangsstoffe für die entsprechenden Carbonsäuren, die durch Verseifung der Trichlormethylgruppe erhalten werden. 1,1,1-Trichlor-4-methylpentanol-4 ist ein Zwischenprodukt für die Pyrethroide Permethrin und Cypermethrin (DE-OS 2 616 528, Nachr. Chem. Tech. Lab. 26, 120 bis 128 [1978]). Bezüglich der Verwendung wird auf die vorgenannten Veröffentlichungen verwiesen.

Die in den folgenden Beispielen genannten Teile bedeuten Gewichtsteile. Die Gewichtsteile verhalten sich zu den Volumenteilen wie Kilogramm zu Liter. Reinheitsgrade der Endstoffe I werden mittels Gaschromatographie bestimmt.

## Vergleichsbeispiel 1

In einem Emailkessel werden 6500 Volumenteile Chloroform unter Rühren auf 130°C erhitzt. Es entsteht ein Druck von 6 bar. In 5 Stunden pumpt man ein Gemisch von 1190 Teilen 1-Octen und 120 Teilen tert.-Butylperbenzoat zu und hält anschließend noch 4 Stunden bei 130°C. Das überschüssige Chloroform wird nach dem Abkühlen bei Normaldruck abdestilliert und der Rückstand im Vakuum destilliert. Bei 78 bis 86°C/0,4 mbar gehen 1757 Teile Destillat mit einem Gehalt von 83 Gew.-% 1,1,1-Trichlornonan über. Ausbeute: 59,3% der Theorie.

### Vergleichsbeispiel 2

In einem Emailkessel werden 6500 Volumenteile Chloroform unter Rühren auf 120° C erhitzt. In 6,5 Stunden pumpt man eine Lösung von 120 Teilen Dibenzoylperoxid in 1190 Teilen 3-Methylbuten-1-ol-3 und 500 Volumenteilen Chloroform zu und hält anschließend das Gemisch noch 4 Stunden bei 120° C. Das überschüssige Chloroform wird nach dem Abkühlen bei Normaldruck abdestilliert und der Rückstand im Vakuum destilliert. Bei 64 bis 68° C/0,4 mbar gehen 1681 Teile Destillat mit einem Gehalt von 79 Gew.-% 1,1,1-Trichlor-4-methyl-pentanol-4 vom Fp. 39 bis 40° C über. Ausbeute: 46,7% der Theorie.

### Vergleichsbeispiel 3

86 Teile 3-Methylbuten-1-ol-3, 8,6 Teile Di-tert.-butylperoxid und 750 Volumenteile Chloroform werden in einem mit Hastelloy C ausgekleideten Rührautoklaven 6 Stunden lang auf 130° C erhitzt. Nach dem Abkühlen destilliert man das überschüssige Chloroform bei Normaldruck und den Endstoff bei 64 bis 68° C/0,4 mbar über. Man erhält 152,1 Teile festes 1,1,1-Trichlor-4-methylpentanol-4 mit dem Reinheitsgrad 77 Gew.-%. Ausbeute: 57% der Theorie.

### Vergleichsbeispiel 4

In einem V2A-Rührkessel werden 9000 Volumenteile Chloroform unter Rühren auf 150° C erhitzt. In einer Stunde pumpt man eine Lösung von 103 Teilen Di-tert.-butylperoxid in 1032 Teilen 3-Methylbuten-1-ol-3 zu und erhitzt das Gemisch noch 10 Stunden auf 150° C. Nach der Aufarbeitung analog Vergleichsbeispiel 2 erhält man 1163 Teile eines nicht kristallisierenden Destillats, das 60 Gew.-% 1,1,1-Trichlor-4-methylpentanol-4 enthält. Ausbeute: 28,3% der Theorie.

### Beispiel 1

In einem Emailkessel werden 6500 Volumenteile Chloroform und 100 Teile wasserfreies Natriumacetat unter Rühren auf 130° C erhitzt. In 5 Stunden pumpt man ein Gemisch von 1190 Teilen 1-Octen und 120 Teilen tert.-Butylperbenzoat zu und hält anschließend das Gemisch noch 4 Stunden bei 130° C. Nach dem Abkühlen werden 2000 Volumenteile Wasser zugesetzt, die wäßrige Phase wird abgetrennt und das überschüssige Chloroform abdestilliert. Bei der anschließenden Vakuumdestillation des Rückstandes erhält man bei 78 bis 86° C/0,4 mbar 2081 Teile Destillat mit einem Gehalt von 85 Gew.-% 1,1,1-Trichlornonan. Ausbeute: 72% der Theorie.

### Beispiel 2

In einem Emailkessel werden 6500 Volumenteile Chloroform und 100 Teile Natriumacetat unter Rühren auf 120° C erhitzt. In 5,5 Stunden pumpt man ein Gemisch von 1190 Teilen 3-Methylbuten-1-ol-3, 120 Teilen Dibenzoylperoxid und 500 Volumenteilen Chloroform zu und hält anschließend noch 4 Stunden bei 120° C. Nach dem Abkühlen werden 2000 Volumenteile Wasser zugesetzt, die wäßrige Phase wird abgetrennt und das überschüssige Chloroform abdestilliert. Bei der anschließenden Vakuumdestillation des festen Rückstandes erhält man bei 64 bis 68° C/0,4 mbar 2384 Teile Destillat mit einem Gehalt von 78 Gew.-% 1,1,1-Trichlor-4-methylpentanol-4 vom Fp. 39° C. Ausbeute: 65,4% der Theorie.

### Beispiel 3

In einem Emailkessel werden 6500 Volumenteile Chloroform und 100 Teile wasserfreies Natriumphosphat unter Rühren auf 120° C erhitzt. In 4 Stunden pumpt man ein Gemisch von 1190 Teilen 3-Methylbuten-1-ol-3 und 120 Teilen tert.-Butylperbenzoat zu und hält anschließend das Gemisch noch 4 Stunden bei 120° C. Nach der Aufarbeitung analog Beispiel 2 erhält man 2235 Teile Destillat mit einem Gehalt von 79 Gew.-% 1,1,1-Trichlor-4-methyl-pentanol-4. Ausbeute: 62,1% der Theorie.

### Beispiel 4

In einem Emailkessel werden 6500 Volumenteile Chloroform und 100 Teile Natriumacetat unter Rühren auf 120° C erhitzt. In 4 Stunden pumpt man ein Gemisch von 1190 Teilen 3-Methylbuten-1-ol-3 und 120 Teilen tert.-Butylperbenzoat zu und hält anschließend das Gemisch noch 4 Stunden bei 120° C. Nach der Aufarbeitung analog Beispiel 2 erhält man 2395 Teile Destillat mit einem Gehalt von 80 Gew.-% 1,1,1-Trichlor-4-methylpentanol-4. Ausbeute: 67,4% der Theorie.

### Beispiel 5

Die Reaktion in Beispiel 4 wird mit 80 Teilen tert.-Butylperbenzoat anstelle von 120 Teilen wiederholt. Man erhält 2245 Teile mit einem Gehalt von 78 Gew.-% 1,1,1-Trichlor-4-methyl-pentanol-4. Ausbeute: 61,6% der Theorie.

### Beispiel 6

In einem Emailkessel werden 6500 Volumenteile Chloroform und 100 Teile Natriumacetat unter Rühren auf 120°C erhitzt. In 4 Stunden pumpt man ein Gemisch von 1190 Teilen 3-Methyl-buten-1-ol-3 und 120 Teilen tert.-Butylperbenzoat zu und hält das Gemisch anschließend noch 8 Stunden bei 120°C. Nach der Aufarbeitung analog Beispiel 2 erhält man 2422 Teile Destillat mit einem Gehalt von 81 Gew.-% 1,1,1-Trichlor-4-methylpentanol-4. Ausbeute: 69% der Theorie.

### Beispiel 7

Die Reaktion in Beispiel 6 wird wiederholt mit dem Unterschied, daß Olefin und Peroxid in 6 Stunden zugepumpt werden und die Mischung anschließend noch 12 Stunden bei 120°C gerührt wird. Man erhält 2524 Teile mit einem Gehalt von 80 Gew.-% 1,1,1-Trichlor-4-methylpentanol-4. Ausbeute: 71% der Theorie.

### Beispiel 8

In einem Emailkessel werden 6500 Volumenteile Chloroform und 100 Teile Natriumacetat unter Rühren auf 130°C erhitzt. In 4 Stunden pumpt man ein Gemisch von 1190 Teilen 3-Methylbuten-1-ol-3 und 120 Teilen Di-tert.-butylperoxid zu und hält anschließend das Gemisch noch 7 Stunden bei 130°C. Nach der Aufarbeitung analog Beispiel 2 erhält man 2614 Teile mit einem Gehalt von 78 Gew.-% 1,1,1-Trichlor-4-methylpentanol-4. Ausbeute: 71,6% der Theorie.

### Beispiel 9

86 Teile 3-Methylbuten-1-ol-3, 8,6 Teile Di-tert.-butylperoxid, 16,8 Teile Natriumhydrogencarbonat und 750 Volumenteile Chloroform werden in einem mit Hastalloy C ausgekleideten Rührautoklaven 6 Stunden lang auf 130°C erhitzt. Nach der Aufarbeitung analog Beispiel 2 erhält man 190 Teile mit einem Gehalt von 77 Gew.-% 1,1,1-Trichlor-4-methylpentanol-4. Ausbeute: 71,2% der Theorie.

### Beispiel 10

Die Reaktion im Vergleichsbeispiel 4 wird unter Zusatz von 87 Teilen Natriumacetat wiederholt. Man erhält 2133 Teile kristallines Destillat mit einem Gehalt von 74 Gew.-% 1,1,1-Trichlor-4-methyl-pentanol-4. Ausbeute: 64% der Theorie.

## Patentanspruch

Verfahren zur Herstellung von 1,1,1-Trichlormethylverbindungen der Formel

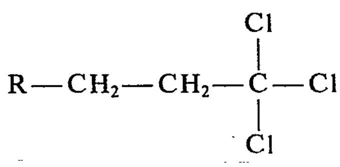

(I)

worin R einen aliphatischen Rest bedeutet, durch Umsetzung von Chloroform mit Olefinen der Formel

$$R-CH=CH_2 \qquad (II)$$

worin R die vorgenannte Bedeutung besitzt, in Gegenwart von Radikalstartern, dadurch gekennzeichnet, daß die Umsetzung in Gegenwart von schwachbasischen Alkali- oder Erdalkalisalzen durchgeführt wird.

## Claim

A process for the preparation of a 1,1,1-trichloromethyl compound of the formula

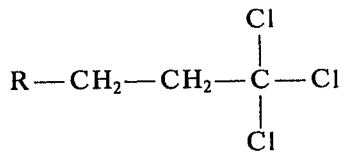

(I)

where R is an aliphatic radical, by reacting chloroform with an olefin of the formula

$$R-CH=CH_2 \qquad (II)$$

where R has the above meaning, in the presence of a freeradical initiator, wherein the reaction is carried out in the presence of a weakly basic alkali metal salt or alkaline earth metal salt.

## Revendication

Procédé de préparation de composés trichloro-1,1,1 méthylés de la formule

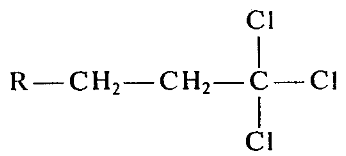

(I)

dans laquelle R désigne un groupe aliphatique, par réaction du chloroforme avec des oléfines de la formule

$$R-CH=CH_2 \qquad (II)$$

dans laquelle R possède la signification définie, en présence d'amorceurs à radicaux libres, caractérisé en ce que la réaction est réalisée en présence de sels de métaux alcalins ou alcalino-terreux faiblement basiques.